(19) Europäisches Patentamt
European Patent Office
Office européen des brevets

(11) Publication number: 0 209 379
A2

(12) # EUROPEAN PATENT APPLICATION

(21) Application number: 86305480.5

(51) Int. Cl.[4]: C07K 1/04

(22) Date of filing: 16.07.86

(30) Priority: 18.07.85 GB 8518144

(43) Date of publication of application:
21.01.87 Bulletin 87/04

(84) Designated Contracting States:
AT BE CH DE FR GB IT LI LU NL SE

(71) Applicant: The Queen's University of Belfast

Belfast BT7 1NN Northern Ireland(GB)

(72) Inventor: Elmore, Donald Trevor
28 Viewpoint Park Dunmurray
Belfast BT17 9JY Northern Ireland(GB)

(74) Representative: Funge, Harry et al
WILSON, GUNN & ELLIS 41-51 Royal
Exchange Cross Street
Manchester M2 7BD(GB)

(54) ## Solid phase peptide synthesis.

(57) A method of solid phase peptide synthesis includes use of a reusable insoluble polymer resin having functional groups of the formula

Resin -$NHRNHCO(CH_2)_nOH$

wherein R is an alkyl or cycloalkyl group having 2 to 20 carbon atoms, an aromatic group, substituted aromatic group, heterocyclic group or substituted heterocyclic group preferably 1,4-dimethylcyclohexane, and N is an integer, preferably 3. Phosphorylation followed by reaction with a diol, preferably 1,4-bis(hydroxymethyl)benzene, affords a substrate upon which peptides may be synthesised by repeated deprotection and coupling with protected amino acids.

EP 0 209 379 A2

## SOLID PHASE PEPTIDE SYNTHESIS

This invention relates to a method of solid phase peptide synthesis and to materials for use in performance of the method.

Use of insoluble polymer resins such as polyamide as a substrate for peptide synthesis is well established. However, currently available resins are expensive and must be discarded after a single use

According to a first aspect of the present invention a method of solid phase peptide synthesis including the steps of:

combining an insoluble polymer resin with a diamine and a lactone; the diamine having the formula (I), wherein R is selected from: an alkyl or cycloalkyl group having 2 to 20 carbon atoms, an aromatic group, substituted aromatic group, heterocyclic group and substituted heterocyclic group;

$H_2NRNH_2$ (I)

and the lactones having the formula

$$\overline{\underline{|}CHX(CH_2)_nCHYCO.O\underline{|}}$$

wherein X and Y are independently selected from: hydrogen, $C_1$ to $C_5$ alkyl or NHZ (where Z is selected from aryl, aroyl or other protecting group) and n is 0,1,2 or 3 to produce an alcohol of formula (II);

Resin $NHRNHCOCHY(CH_2)_nCHXOH$ (III)

phosphorylating the alcohol (II) with an arylphosphorodichloridate to produce an alkyl aryl phosphorochloridate;

treating the alkyl arylphosphorochloridate with a compound of formula (III) wherein l and m are integers from 1 to 4, and $R_1$ is a cycloalkyl group, aromatic group, substituted aromatic group, heterocyclic group or substituted heterocyclic group to produce a phosphotriester;

$HO(CH_2)_lR_1(CH_2)_mOH$ (III)

treating the phosphotriester with base to produce a dialkylphosphate ester of formula (IV)

$$Resin\text{-}HN\text{-}R\text{-}NHCOCHY(CH_2)_nCHXO\overset{\overset{\displaystyle O}{\|}}{\underset{\underset{\displaystyle O^-}{|}}{P}}O(CH_2)_lR_1(CH_2)_mOH \quad (IV)$$

condensing the dialkylphosphate ester (IV) with a protected amino acid anhydride;

forming a peptide by repeating a process of deprotection and coupling with protected amino acids,

treating the peptide with phosphodiesterase to cleave the peptide from the resin.

The free peptide may be obtained by removal of the diol(III) by catalytic hydrogenolysis, treatment with strong acid or by use of an appropriate enzyme.

The resin resultant from the treatment with phosphodiesterase may be treated with alkaline phosphatase to produce the alcohol(II) which can be reused. The cleaved peptide derivative can also be treated with alkaline phosphatase to give a peptide ester. A peptide amide can be obtained by cleavage of the peptide from the resin with ammonia.

Initial treatment of the insoluble resin with the compound (I) serves to distance the subsequently formed phosphate from the surface of the resin facilitating hydrolysis by phosphodiesterase which could otherwise be sterically inhibited. Preferred compounds (I) have a rigid carbon skeleton such as cycloaliphatic or other rings.

The diamine and lactone are preferably condensed together to form a compound of the formula (V) wherein R and n are as defined above.

$H_2NRNHCOCHY(CH_2)_n CHXOH$ (V)

The compound (V) may be conveniently condensed with the resin to produce the compound - (II). Alternatively the diamine (I) may be condensed with the resin and the product in turn condensed with the lactone to produce the compound (II). Combination of the resin and compound (V) is preferred to minimise side reactions,thereby providing a cleaner product having superior physical properties.

A particularly preferred diamine (I) is bis-(aminomethyl)cyclohexane. The 1,3-or 1,4-isomers may be employed.

Preferred lactones have n = 1 and one of X or Y being hydrogen the other being hydrogen or methyl.

Especially preferred lactones are γ-butyrolactone or a protected homoserine lactone i.e. wherein n is 3, for example N-acetyl homoserine lactone.

4-Nitrophenylphosphorodichloridate has been found to be a suitable phosphorylating agent. Other agents which have labile leaving groups may be employed alternatively.

A preferred diol (III) is 1,4-bis(hydroxymethyl) benzene but other 4-substituted benzyl alcohols are also preferred. 1, 3-bis(hydroxymethyl)benzene may also be used but is more expensive. An alternative reagent is bis(hydroxymethyl)furan.

Use of a polyacrylamide resin is preferred although other resins known to those skilled in the art e.g. polystyrene may be employed.

The protected amino acid anhydride is preferably the N-fluorenylmethoxycarbonyl (Fmoc) derivative, although the t-butyloxycarbonyl (Boc), O-tertiary butyl or 4-methoxy-2,3,6-trimethylbenzenesulphonyl (Mtr) derivatives may be used alternatively.

According to a second aspect of the present invention a solid phase peptide synthesis substrate comprises a functionalised insoluble polymer resin having the formula (II) wherein R, X, Y and n are as previously defined.

The invention is further described by means of example and not in any limitative sense, with reference to the following example.

## EXAMPLE

Preparationof 1-N-(4'-hydroxybutanoyl)aminomethyl-3-aminomethyl cyclohexane.

1,3-Bis (aminomethyl)cyclohexane (30g. 0.21 moles) and γ-butyrolactone (18g 0.21 moles) were stirred together. After about 30 min, the flask became warm and after several hours, the mixture was extremely viscous. Dioxan (50ml) was added and the mixture was heated to approximately 80°C for 6 h with stirring. The bulk of the solvent was removed in a rotary evaporator and the remainder and any unchanged diamine was removed at <1mm pressure at 100°C. The product (45g) was a viscous oil which was homogeneous on thin-layer chromatography and could be used without further purification.

Derivatization of polyacrylamide resin

A polyacrylamide resin with functional ester groups (e.g. "Pepsyn K", Cambridge Research Biochemicals, Cambridge, UK) (2.0g) was suspended in NN-dimethyl-formamide (50ml) and 1-N-(4'-hydroxanoyl)aminomethyl-3-aminomethyl-cyclohexan e (10 g) was added. The mixture was agitated at 30°C for 4 days and the resin was washed 10 times with 20 ml aliquot of NN-dimethyl-formamide. A small portion of the product resin was tested for the absence of free amino groups by the ninhydrin reagent.

The foregoing resin derivative was treated with p-nitrophenyl phosphorodichloridate (5g) in NN-dimethylformamide (25ml) followed by pyridine - (25ml) and the mixture was agitated under nitrogen for 27 h during which time the supernatant turned from pale red to almost black. The supernatant was removed and the resin was washed by resuspension once with pyridine -NN-dimethylformamide (40 ml) 1:1 by volume) and five times with NN-dimethylformamide (25 ml). The product resin turned a golden yellow colour as a result of the phosphorylation step.

The product from the previous step was treated with a solution of 1,4-bis(hydroxymethyl) benzene in NN-dimethylformamide (20 ml and pyridine (20 ml) with agitation for 40h. The supernatant was removed and the product resin was washed by resuspension once with pyridine -NN-dimethylformamide mixture and five times with NN-dimethylformamide.

The product from the previous step was suspended for 3h in a mixture of NN-dimethylformamide (20 ml), piperidine (20 ml) and water (20 ml) in order to hydrolyse the phosphotriester. The resultant resin was washed by resuspension twice in the above solvent, twice with NN-dimethylformamide -piperidine (1:1 by volume), five times with NN-dimethylformamide, twice with NN-dimethylformamide -methanol(1:1 by volume), five times with methanol, twice with methanol -dichloromethane - (1:1 by volume) and five times with dichloromethane. The derivatized resin was filtered and dried over $P_2O_5$ and silica gel in a desiccator.

Procedure for typical syntheses of peptides using a phosphate-type matrix.

Coupling steps were generally carried out with symmetrical anhydrides of 9-fluorenylmethoxy carbonyl (Fmoc) protected amino acids in NN-dimethylformamide using a 2-6 fold excess of anhydride. Where required, functional side chains of Fmoc-amino acids were protected with appropriate groups such as N-tert-butoxycarbonyl (Boc), O-tert-butyl, 4-methoxy-2,3,6-trimethylbenzenesulphonyl - (Mtr). In the case of Fmoc-asparagine and Fmoc-glutamine, coupling was achieved using a reactive ester (e.g. p-nitrophenyl) in presence of 1-hydroxybenzotriazole in NN-dimethylformamide. After completion of each coupling step, the Fmoc-protecting group was removed by exposure to piperidine in NN-dimethylformamide. Alternatively, the peptide could be assembled using Boc-amino acids.

After completion of the assembly of the peptide on the resin, all protecting groups could be removed by standard procedures or left intact for further coupling to other peptides by classical solution methods

Procedures for detatchment of peptides from a phosphate-type matrix.

The peptide could be recovered as a C-terminal amide by treatment with methanol saturated with ammonia. The resin was reusable after washing with NN-dimethylformamide.

The peptide could be recovered as a C-terminal p-hydroxymethylbenzyl ester by a two-stage enzymic cleavage process.

The resin was first exposed to phosphodiesterase (e.g. from spleen) in 0.15M sodium acetate buffer (pH5.0) containing 0.01M ethylenediamine tetraacetate. It was important to inhibit irreversibly any contaminating proteinases, for example, by prior treatment with iodoacetamide. The second cleavage step was achieved by exposure to alkaline phosphomonesterase at pH10. The recovered resin could be recycled by entering the derivatization process at the stage involving phosphorylation with p-nitrophenyl phosphorodichloridate. If recycling of the resin was not essential, only the first cleavage step with phosphodiesterase was necessary.

Irrespective of whether phosphodiesterase was used alone or followed by treatment with phosphomonesterase, the peptide with a free terminal carboxyl group could be obtained by cleavage of the substituted benzyl ester function using 33% HBr in acetic acid.

In all cases it was desirable to subject the isolated peptide to purification, for example, by high-performance liquid chromatography.

Using the above methodology, peptides such as Leu$^5$-enkephalin, casomorphin and substance P of high purity have been synthesized in good yield.

## Claims

1. A method of solid phase peptide synthesis including the steps of:

combining an insoluble polymer resin with a diamine and a lactone; the diamine having the formula (I), wherein R is selected from: an alkyl or cycloalkyl group having 2 to 20 carbon atoms, an aromatic group, substituted aromatic group, heterocyclic group and substituted heterocylic group;

$H_2NRNH_2$ (I)

and the lactone having the formula

$$\overline{\mathrm{CHX(CH_2)_nCHYCO.O}}$$

wherein X and Y are independently selected from:

hydrogen. $C_1$ to $C_5$ alkyl or NHZ (where Z is selected from aryl, aroyl or other protecting group) and n is 0, 1,2 or 3.

Resin -NHRNHCOCHY$(CH_2)_n$CHXOH (II)

phosphorylating the alcohol (II) with an arylphosphorodrichloridate to produce an alkyl aryl phosphorochloridate;

treating the alkyl arylphosphorochloridate with a compound of formula (III) wherein l and m are integers from 1 to 4, and $R_1$ is a cycloalkyl group, aromatic group, substituted aromatic group, heterocyclic group or substituted heterocyclic group to produce a phosphotriester;

$HO(CH_2)_l R_1(CH_2)_mOH$ (III)

treating the phosphotriester with base to produce a dialkylphosphate ester of formula (IV)

$$Resin-HN-R-NHCOCHY(CH_2)_nCHXO\underset{O^-}{\overset{O}{\overset{\|}{P}}}O(CH_2)_1R_1(CH_2)_mOH \quad (IV)$$

condensing the dialkylphosphate ester (IV) with a protected amino acid anhydride;

forming a peptide by repeating a process of deprotection and coupling with protected amino acids,

treating the peptide with phosphodiesterase to cleave the peptide from the resin.

2. A method of solid phase peptide synthesis as claimed in claim 1, including the steps of:

condensing the diamine of formula (I) with the lactone of formula

$$\overline{CHX(CH_2)_nCHYCO.O}$$

wherein X and Y are independently selected from: hydrogen, $C_1$ to $C_5$ alkyl or NHZ (where Z is selected from aryl, aroyl or other protecting group) and n is 0, 1, 2 or 3 and condensingthe product with the insoluble polymer resin.

3. A method as claimed in claim 1 or 2 wherein the diamine is selected from: 1,3-and 1,4-bis-(aminomethyl) cyclohexanes.

4. A method as claimed in any preceding claim wherein the lactone is -butyrolactone.

5. A method as claimed in any of claims 1 to 3 wherein the lactone is a protected homoserine lactone.

6. A method as claimed in any preceding claim wherein the diol is 1,4-bis(hydroxymethyl) benzene.

7. A method as claimed in any preceding claim wherein the peptide is cleaved from the resin by treatment with phosphodiesterase and alkaline phosphatase.

8. A solid phase peptide synthesis substrate comprising a functionalised insoluble polymer resin having the formula (II) wherein R, X, Y and n are as previously defined.